# EUROPEAN PATENT SPECIFICATION

(11) **EP 1 455 772 B1**
(45) Date of publication and mention of the grant of the patent: **29.07.2009**
(21) Application number: 02713949.2
(22) Date of filing: 08.04.2002
(51) Int. Cl.: A61K 31/19, A61P 35/00, A61P 31/10, A61P 17/00

(54) **MEDIUM FOR RADICAL TREATMENT OF TUMORS, INFLAMMATORY PROCESSES, FUNGAL INFECTIONS AND OTHER AFFECTIONS OF THE TISSUES, DIAGNOSTIC AND PROPHYLACTIC AND METHOD OF ITS IMPLEMENTATION**
MEDIUM ZUR RADIKALEN THERAPIE VON TUMOREN, VON ENTZÜNDUNGSPROZESSEN, VON PILZINFEKTIONEN UND VON ANDEREN AFFEKTIONEN DER GEWEBEN; DIAGNOSTISCHE UND PROPHYLAKTISCHE VERFAHREN SOWIE DEREN IMPLEMENTIERUNG
MILIEU DESTINE AU TRAITEMENT RADICAL DES TUMEURS, DES PROCESSUS INFLAMMATOIRES, DES INFECTIONS FONGIQUES ET D'AUTRES AFFECTIONS DES TISSUS, PROCEDES DIAGNOSTIQUE ET PROPHYLACTIQUE ET PROCEDE DE MISE EN OEUVRE DUDIT MILIEU

(30) Priority: 12.12.2001 BG 10621501
(43) Date of publication of application: 15.09.2004
(73) Proprietor: Panchev, Vladimir, 1324 Sofia (BG); Pancheva, Marieta, 1303 Sofia (BG); Souvandjieva, Adelina, 1303 Sofia (BG)
(72) Inventor: Panchev, Vladimir, 1324 Sofia (BG); Pancheva, Marieta, 1303 Sofia (BG); Souvandjieva, Adelina, 1303 Sofia (BG)
(86) International application number: PCT/BG2002/000006
(87) International publication number: WO 2003/049728

(56) References cited:
- EP-A- 0 259 617
- WO-A-97/41434
- US-A- 4 927 813
- US-A- 5 462 714
- US-B1- 6 231 840
- N.A. WOOD: "Treatment of Established Infections of Candidasis in Partridges with Formic Acid" THE VETERINARY RECORD, vol. 87, no. 21, 1970, pages 656-658, XP001096030
- RAMESH M. BHAT ET AL.: "Topical formic acid puncture technique for the treatment of common warts" INTERNATIONAL JOURNAL OF DERMATOLOGY, 2001, pages 415-419, XP001097776
- NAIK R B ET AL: "Ingestion of formic acid-containing agents--report of three fatal cases." POSTGRADUATE MEDICAL JOURNAL, (1980 JUN) 56 (656) 451-6. , XP001096025

## Description

### Background of the invention

The known methods of threatening tumors for the time being are the followings:
1) Surgical method, including electro-diathermy-surgical and laser-surgical intervention
2) Radiating methods of treatment, including, in line with the roentgen rays, the use of radio and radioisotope radiation.
3) Medicine methods, including chemytherapeutic mediums, antibiotics and hormones.
4) Different combinations of the above- mentioned methods.
Taking into account that the cancer illnesses take second place in the worlds range of mortality, after the heart-vascular illnesses, and the cruel consequences for the health and the psychological condition of the patients from the basically applied - surgical and radiation methods of treatment, every innovation in this field should have an enormous significance for the development of the medicine and consequently for the people's life.

The diagnostic of the tumors for the time being is effectuated through the habitual methods of clinical examination of the patients, including X-rays, echological, endoscopical and cytological examinations. In dependence from the localization of the tumor are implemented different methods of examination. Independently from the great number of proposals concerning the biochemical, physicochemical and other methods of laboratory diagnostic without tacking tissue, no one of the proposed reactions "of cancer" can still be considered for a specific and more or less reliable and the diagnosis is to be made on the basis of comprehensive clinical examination of the patient. These faults of the background in the field of diagnosing the tumors manifest themselves especially strongly by the early stages of cancer, the correct diagnosis of which is exclusively important for the effective treatment. In this condition the cancer neo-formations are very difficult to be sensed and in the majority of the cases do not cause painful reactions.

### Description of the invention

The proposed medium and method for radical treatment of tumors is based on the following general principals:
1. Atypicity of the tumor tissue, distinguishing it from the healthy one in direct proportion of its malignity and especially of the atypicity of its blood vessels. For an example, the atypical, often lagoon-like vessels of the malignant tumors sometimes consist of thin layer endothelia, while on places the tumor cells are in a direct touch with the washing and nourishing the tumor blood and lymph fluid.
2. The considerably better protection of the blood vessels of the healthy tissue, having three-layer cover, two of the layers of which are nourished by self blood vessels (vasa vasorum).
3. The property of the formic (methane) acid (HCOOH) to cause blood coagulation, probably destroying the anticoagulants in it.
4. The fact that the formic acid is contained in human's and animal's organism and participates actively in its biochemical processes reduces to a minimum the risk from side effects during the treatment, in comparison with the most of the other methods in use.
5. The simplicity of its molecule, as the mentioned in the above point 4, are ideal premises for the rapid soaking of its water solutions in the organism. This allows the treatment of internal tumors with such a solution as well, without the help of a syringe, sound or surgery intervention. These are some of its most characteristic properties:
   - It is the most powerful among the all carbonic (organic) acids;
   - Is the simplest of them;
   - It is the only one from the carbonic acids, which gives reaction of acid and aldehide as well, due to the fact that the hydroxyl carbon in it is bounded not to a radical, but to the 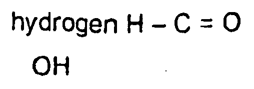
   - as an aldehid it is a powerful restorator;
   - it has very strong expressed coagulative effect on the blood;
   - it has strong bactericide and common antiseptic effect.

### References:

**D1:** US-B1-6 231 840 (BUC CAROL J) 18 May 2001 **(**2001-06-16**) (column 20, claims 1, 2, 4, 12)** refers to a pharmaceutical composition to the treatment of mammalian nail fungal conditions comprising an alkanoic acid, including methanoic, in ranges greater than 5% and between 8% and 20% by weight. In this patent are used the properties of alcanoic acids to denaturing keratin and on that base the application for treatment of hyperkeratotic tissue, including such, caused by fungal infection.
**D2:** EP-A.O 269 817 (CHIMICASA GMBH) 18 March 1988 **(4988-03-16)** is based on the antiviral action of the formic acid.
**D3:** US-A-4 927 813 (BERNSTAIN JOEL E) 22 May 1990 (1990-05.22) relays to a composition and method for removing head lice nits from a human by applying therapeutically effective amount of formic acid.
**D4:** N.A. WOOD: "Treatment of Established Infections of Candidasis in Partridges with Formic Acid" THE VETERINARY RECORD, vol. 87, no. 21 1970, pages 656-658**, XP001096030** refers to the treatment of Candida albicans infections in partridge chicks, using 6%, 10% and 15% of formic acid.
**D6:** US-A-5 462 714 (TALWALKER RAMESH T **ET AL) 31 October 1998-10-31)** refers to antimicrobial agents comprising 0,25%-2% iodine and 20%-50% fatty acid, including formic acid.
**D6:** WO 97/41434 A **(PEREGRIN PHARMACEUTICAL INC)** 6 November 1997 **(1997-11-06).** The object of this application is to develop a method for determining whether a mammal should be medically investigated for a cancerous or a pre-cancerous condition.
**D7:** RAMESH M. BHAT ET AL.: "Topical formic acid puncture technique for the treatment of common warts" INTERNATIONAL JOURNAL OF DERMATOLOGY, 2001, PAGES 415-419**, XP001097776** refers to use of 85% formic acid for treatment of common warts.
**D8:** NAIK R B ET AL: "Ingestion of formic acid-containing agents-report on three fatal cases." POSTGRADUATE MEDICAL JOURNAL, (1980 JUN) 56 (656) 451-6**, XP001096025**

### Principe of the method

Consolidating the above mentioned in points 1, 2 and 3, 4 and 5 principles, around the common target - to destroying the tumor formations, with minimum risk for the patient's health and maximum effectiveness, the present invention utilizes for the treatment of tumors a water solution of formic acid, to which can be added a little iodine (for accelerating its soaking by the tissues), with different concentration and respectively power of action. The implementation of the respective variant depends on the concrete case and on the qualification of the treating physician as well. As a general rule, by the maligns tumors is sufficient weaker solution - about 10 %, which is an exceptional advantage, taking into account their possible infiltration and with a view to protect the healthy tissue, considering that in concentrated form the formic acid has a caustic effect on tissues. The goal is that the tissues are gradually saturated with it, by which on the first place will be necrotized the tumor ones.

The treatment is provided by rub in the medium with a tampon impregnated with it, directly in the tumor (by the skin ones), or in the accessible surface of the healthy tissue situated near by it. The rub in takes place repeatedly, with waiting for the absorption of the solution, until the tumor is saturated with formic acid in such a degree, that the blood in its blood-vessels coagulates and so that the tumor will be necrotized. As the increasing of the concentration of formic acid in the both sorts of tissue (the healthy and the tumor ones) goes gradually, the necrotisation of the tumor tissue, due to the enumerated in the above point 1 specificity of its blood-vessel system, occurs before the concentration of formic acid in the healthy tissue could reach damaging levels for it. This makes the method ideal in combating each sort of tumor formations, independently of their malignity, stage of infiltration or location. On the contrary, as much a tumor is malignant, the principles on which lies the invented method lead to the logical assumption that it is more vulnerable, which is practically confirmed. By the benign tumor the treatment shall include mechanical irritation of its tissue too, with the purpose to stimulate the blood-circulation in it, by which the blood- vessels will be filled out and their surface will be enlarged on the account of the thickness of their walls. Such a treatment can be utilized, if necessary, by the malignant tumors too.

By the implementation of the invention a tribute must be paid not to overpass the limited concentration of formic acid in the organism, which can lead to poisoning. Primary signs of such an overpass are the appearance of a specific taste in the mouth, bad feeling and in more strong degree - to head turning. Interruption in the proceedings for twenty-four hours, combined with drinking of more liquids and by necessity diuretants too, is satisfactory for putting aside these phenomena, because the formic acid is to be exuded through the urine and the sweat.

With the view of preventing the above described side phenomena, the treatment of bigger and numerous tumors shall be provided sequentially, on areas not bigger than 30 - 40 sq. sm., as the duration of one seance is in reverse proportion to the treated surface.

Until now the invention is repeatedly implemented for the elimination of benign human skin tumors through rub in. Also, only through rub in, it was successfully implemented for treatment of malign formation in the milk gland with dimensions 5,7 to 7,4 mm. and about 8 mm. deep. Considering that only a few minutes after the beginning of the rub in, on this deep begins a process of necrotisation of the tumor tissue, can be concluded that on greater deepness and by other organs a treatment through rub in is possible too, without necessity of direct supplying of the solution to the tumor.

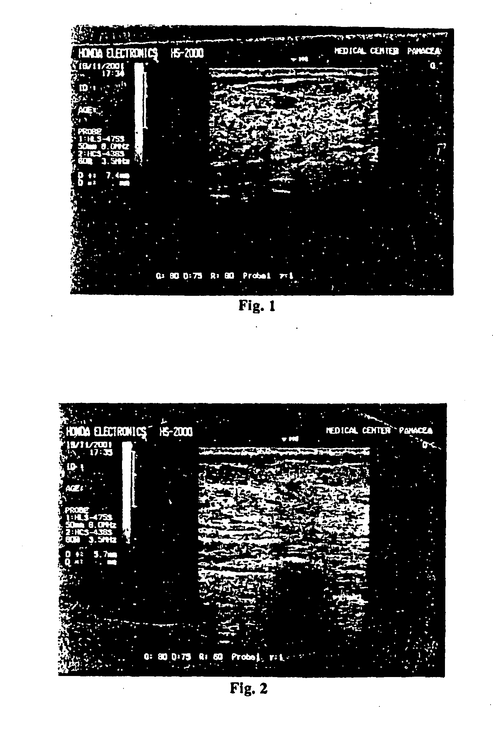

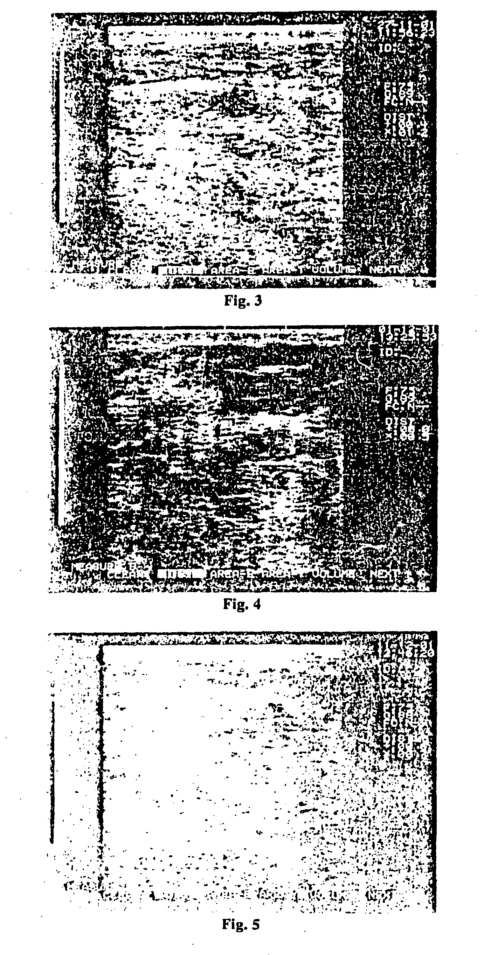

In the concrete case the treatment is applied on the spouse and respectively mother of the inventors. The women is 52 years old, with two side fibrose mastopatia. On 19.11.2001 by an echographic examination has been discovered malign formation in the left breast, sector 12h., with the above mentioned dimensions and deepness, (Fig.1 and Fig. 2). The diagnosis was confirmed through punctual biopsy. The treatment by the above-mentioned method began on 23.11.2001 in the evening. Several minutes after the beginning of the rub in of the medium, a strong internal ache started, which continued the entire night. The procedures continued three times daily in the following days. During the next day and night the aches continued. After that they stopped, notwithstanding that the procedures continued about 10 days more. On the echographic examinations, made on 27.11.2001, 01.12.2001 and 11.12.2001, showed respectively on Fig. 3, Fig. 4 and Fig. 5 could be monitored the change in the configuration, the dimensions and the density of the flack. When making the echographya, given on fig.4 has also been made a second punction biopsy, by which have not been observed cancer cells. The analyze of the tomograms of the flack, given on fig. 5 shows, that the flack is diminished, and is divided on two parts, with common approximation to the breast surface. The nature of this dynamic, combined with the results of the biopsy and the lack of pain by the rub in of the medium, shows that the tissue in question is necrotized and is dissolved in the surrounding tissue.

The invention and the principals on which it is based open unlimited possibilities for tumor treatment in other organs in the human's body too, different from the above cases, and namely:
- through inhalations - for the respiratory tract, including the lungs;
- through gradually supply of the medicine in the tumor neighboring tissue, and by need in it, through punction, as in all cases is recommendable that the treatment begins from the surface of the tumor tissue. In case that a break of its integrity is necessary, even through punction only, this shall be applied after the necrotisation of its parts laying in touch with the healthy tissue.
- by an urgent need, through surgical reaching the tumor and its saturation with the invented medicine, as in the most of the cases should be not necessary the mechanical removal of the necrotised tissue, due to the ability of the organism to dissolving and absorbing it alone. In the cases where, despite of all, it is necessary to remove the tumor mechanically, this must be done after its complete necrotisation, aiming to prevent metastasis. In such cases, the presence of the medium in the neighboring to the tumor healthy tissue, acting to closing all open blood vessels and stark bactericide gives supplementary warranty against the invasion of cancer sells through the blood system and the formation of metastasis

### Priorities of the invention

1. By developing of enough precise technology for supplying the medium to the most inaccessible organs of the body, which could not represent a problem, would ensure 100 % favorable outcome of the treatment, supposing that to the moment of detection of the tumor have not been damaged life-important functions of the touched organ.
2. Gives 100% warranty against invasion of tumor cells in the surrounding tissue, the blood and the lymph systems as a result of the treatment itself.
3. From now on could estimate completely non surgery treatment of all skin tumors, the under-skin tumors, the mammary tumors, the thyroid gland, the under-skin lymph nods and in general all organs, to which surface can be approached at least from 1 -2 sm. distance.
4. Fully harmless for the rest tissues and for the organism as a whole, by observing the above given recommendations for conducting the treatment.
5. Incomparably more painless.
6. Does not leave seeable traces from the treatment.
7. May be the only method by which the rapidity of the treatment is in direct proportion to the malignity of the tumor, understandably by the same accessibility of the damaged organ.
8. By the rapidity of action can be compared only with the surgical method, due to which is named "radical".
9. Incredibly cheap.
10. Will free the humanity from the nightmare of the cancer.

### Treatment of inflammatory processes, mushroom formations, and other pathological alterations of the tissues, blood-stopping.

By similar means, through rub in, pulverizing, inhalations, or other methods of supplying to the damaged place, the medium can be applied for treatment of every kind of inflammatory processes, external and internal and for blood - stopping as well. As an example could be given: skin exemes, pus furuncles, dandruf, inflammations of the mouth vetricle, the larynx, the gingivas, the sinuses, dental grenolom, inflammations of the joints and of the nerves.

In each one of the enumerated areas the invention acts very effectively and rapidly. The reaching of the medium to the internal parts of the body, combined with its rapid action gives it big priorities comparably to the until now known medicines for local implementation.

It has been implemented many times for all above-mentioned illnesses. By the three cases of implementation for tooth grenolom the extraction of the teeth was prevented.

### Diagnostic and prophylactic

Our studies show that even very weak solutions of formic acid - in the range of 10 % - give pain reaction when hitting places with pathologically altered tissue, the same being of tumor, inflammatory, mushroom, or other nature. Based on this can be provided diagnostic and prophylactic of these diseases, implementing the same procedure and the above enumerated methods of supplying the medium to the examined place, as by the treatment. By broader studies can be composed a table for the painfulness of the reaction by the different groups of tumors and other types of tissue alteration, depending on the solution concentration, the time of influence, for example by rub in and the deepness of the alteration.

Until these dependencies could be fully established, it is recommended a supplementary examination of the place of the painful reaction with the known methods (rentgenological, echological, punction and, etc.) In principal, they should establish the presence of the necrotized altered tissue, as the diagnostic or prophylactic normally includes the simultaneous treatment of the disease too.

The method has been applied for diagnostic of breast cancer.

### Priorities of the implementation of the invention for diagnostic and prophylactic

1. Security -the exact place of alteration of tissue could be established, even by hard ascertainable neoplasm in a very initial stage, i.e. the risk of oversight, which exists by the other methods, could be eliminated.
2. Simple and easy for implementation.
3. Economical.

### Literature: Great Medical Encyclopedia (USSR)

Enclosure: 1 sheet - 5 echogr. Fig.

## Claims

1. Composition comprising a water solution of formic acid for use in a radical treatment, diagnosis and prophylaxis of benign and malignant tumors, wherein the formic acid is to be supplied to the damaged place.

2. Composition for use according to claim 1, wherein the composition contains about 5% iodine.

3. Composition for use according to claim 1 or 2, wherein the treatment, diagnosis and prophylaxis is based on the different permeability of the blood vessels of the tumor and the healthy tissues against the influence of blood coagulation enhancing substances.

4. Composition for use according to any preceding claim, wherein the tumor can be approached at least from 1-2 cm distance.

5. Composition comprising a water solution of formic acid for use in a treatment, diagnosis and prophylaxis of each type of inflammation, external and internal, for blood-stopping, skin eczema, pus furuncles, dandruff, inflammation of the mouth cavity, gingival, the larynx, the sinuses, dental granuloma, inflammation of the joints and nerves, wherein the formic acid is to be supplied to the damaged place through rub in, inhalation or spraying or any other method of supplying the medicine to that place.

6. Composition for use according to claim 5, wherein the composition contains about 5% iodine.

## Patentansprüche

1. Mischung, bestehend aus Wasserlösung der Ameisensäure zur Verwendung bei radikaler Behandlung, Diagnostik und Prophylaxis von gut- und bösartigen Tumoren, wobei die Ameisensäure bis zur beschädigten Stelle geliefert werden muss.

2. Mischung zur Verwendung nach Anspruch 1, wobei die Mischung ca. 5% Jod enthält.

3. Mischung zur Verwendung nach Anspruch 1 oder 2, wobei die Behandlung, Diagnostik und Prophylaxis sind basiert auf der verschiedenen Durchlässigkeit der Tumorblutgefässe und des unbeschädigten Gewebes gegen die Einwirkung von Mitteln, welche die Blutgerinnung erhöhen.

4. Mischung zur Verwendung nach den oben aufgeführten Ansprüchen, wobei die beschädigte Stelle mindestens from 1-2 cm erreicht werden kann.

5. Mischung, bestehend aus Wasserlösung der Ameisensäure zur Verwendung bei Behandlung, Diagnostik und Prophylaxis von jeder Art inneren und ausseren Entzündungen, zur Blutstillung, bei Hautekzem, Eiterfurunkel, Schuppen, Mund-, Zahnfleisch-, Laryngs- und Sinusentzündung, Zahngranulom, Gelenk- und Nervenentzündung, wobei die Ameisensäure bis zur beschädigten Stelle geliefert werden muss, entsprechend, durch Einreiben, Inhalation oder Spray oder durch jede andere Methode zur Lieferung der Ameisensäure bis zu dieser Stelle.

6. Mischung zur Verwendung nach Anspruch 5, wobei die Mischung ca. 5% Jod enthält.

## Revendications

1. Mixtion incluant solution d'eau d'acide formique pour être utilisée dans un traitement radical, diagnostique et prophylaxie de tumeurs bénignes et malignes ou l'acide formique doit être fournie à la place abîmée.

2. Mixtion à utiliser conformément revendication 1 où la mixtion contient environ 5% d'iode.

3. Mixtion à utiliser conformément revendication 1 ou 2 où le traitement, la diagnostique et la prophylaxie sont basés sur la différente perméabilité des vaisseaux sanguins de la tumeur et des tissus sains contre l'influence des substances qui augmentent la coagulation du sang.

4. Mixtion à utiliser conformément chacune des revendications précédentes où la place abîmée est rapprochée au moins de 1-2 cm distance.

5. Mixtion incluant de solution d'eau d'acide formique pour traitement, diagnostique et prophylaxie de tout type d'inflammations, internes et externes, hémostase, eczéma, abcès, pellicule, infection de la cavité orale, gencives, larynx, sinus, granulome dentaire, inflammation articulaire et des nerfs, où l'acide formique doit être fournie à la place abîmée par friction, inhalation ou injection ou chaque autre méthode pour fournir le médicament à cette place.

6. Mixtion à utiliser conformément revendication 5 où la mixtion contient environ 5% d'iode.
